# EUROPÄISCHE PATENTSCHRIFT

(11) **EP 0 679 080 B1**
(45) Veröffentlichungstag und Bekanntmachung des Hinweises auf die Patenterteilung: **05.07.2000**
(21) Anmeldenummer: 95900714.7
(22) Anmeldetag: 11.11.1994
(51) Int. Cl.: A61K 31/195

(54) **L-ARGININ UND ANALOGE ALS THROMBOZYTENAGGREGATIONSHEMMER**
L-ARGININE AND ANALOGUES AS THROMBOCYTE AGGREGATION INHIBITORS
L-ARGININE ET ANALOGUES UTILISES COMME INHIBITEURS DE L'AGREGATION PLAQUETTAIRE

(30) Priorität: 12.11.1993 DE 4338793
(43) Veröffentlichungstag der Anmeldung: 02.11.1995
(73) Patentinhaber: Frölich, Jürgen C., Prof. Dr. med., 31832 Springe-Lüdersen (DE)
(72) Erfinder: Frölich, Jürgen C., Prof. Dr. med., 31832 Springe-Lüdersen (DE); Böger, Rainer H., 30880 Laatzen (DE)
(74) Vertreter: VOSSIUS & PARTNER
(86) Internationale Anmeldenummer: EP9403741
(87) Internationale Veröffentlichungsnummer: WO9513060

(56) Entgegenhaltungen:
- EP-A- 0 441 119
- EP-A- 0 546 796
- DE-A- 4 305 881
- NUTRITION (UNITED STATES), SEP-OCT 1992, VOL. 8, NO. 5, PAGE(S) 371, Das UN 'L-arginine, nitric oxide and collagen vascular diseases: a potential relationship? [letter]'
- Z KARDIOL (GERMANY), 1991, VOL. 80 SUPPL 5, PAGE(S) 3-6, Schror K 'Endotheliale Faktoren und Thrombozytenfunktion.'
- ENDOTHELIUM, Bd.1, Oktober 1993 Seite S84 R.H. BÖGER ET AL. 'Intravenous L-arginine induces nitric oxide formation and increases peripheral arteriolar blood flow in humans' in der Anmeldung erwähnt
- AM J PHYSIOL (UNITED STATES), NOV 1992, VOL. 263, NO. 5 PT 2, PAGE(S) H1632-5, MORIKAWA E ET AL 'L-arginine decreases infarct size caused by middle cerebral arterial occlusion in SHR.'
- BR J PHARMACOL (ENGLAND), DEC 1992, VOL. 107, NO. 4, PAGE(S) 905-7, MORIKAWA E ET AL 'L-arginine dilates rat pial arterioles by nitric oxide-dependent mechanisms and increases blood flow during focal cerebral ischaemia.'
- INT J CLIN LAB RES (GERMANY), 1991, VOL. 21, NO. 2, PAGE(S) 202-3, AGOSTONI A ET AL 'L-arginine therapy in Raynaud's phenomenon?'
- N ENGL J MED (UNITED STATES), JAN 9 1992, VOL. 326, NO. 2, PAGE(S) 90-4, RAJFER J ET AL 'Nitric oxide as a mediator of relaxation of the corpus cavernosum in response to nonadrenergic, noncholinergic neurotransmission.'
- INT J IMPOT RES (ENGLAND), MAR 1994, VOL. 6, NO. 1, PAGE(S) 33-5;DISCUSSION 36, ZORGNIOTTI AW ET AL 'Effect of large doses of the nitric oxide precursor, L-arginine, on erectile dysfunction.'
- CARDIOSCIENCE (ITALY), SEP 1991, VOL. 2, NO. 3, PAGE(S) 161-5, DONI MG ET AL 'Nitrovasodilators and cGMP inhibit human platelet activation.'
- 26TH ANNUAL MEETING OF THE ASN (AMERICAN SOCIETY OF NEPHROLOGY), BOSTON, MASSACHUSETTS, USA, NOVEMBER 14-17, 1993.;& J AM SOC NEPHROL,, VOL. 4, NO. 3, PAGE(S) 960, 1993. RUILOPE L M ET AL 'EFFECT OF IV L ARGININE L-ARG ON RENAL FUNCTION IN RENAL TRANSPLANT PATIENTS TX TREATED WITH CYCLOSPORINE CYA'
- CIRCULATION (UNITED STATES), APR 1994, VOL. 89, NO. 4, PAGE(S) 1615-23, DREXLER H ET AL 'Effect of L-arginine on coronary endothelial function in cardiac transplant recipients. Relation to vessel wall morphology.'

## Beschreibung

Die Erfindung betrifft den Einsatz von L-Arginin und seinen Derivaten als Thrombozytenaggregationshemmer zur Behandlung der Transplantatabstoßung, der Impotentia coeundi von Haarwuchsstörungen Diese Verbindungen sind in der Lage, die Symptome dieser Erkrankungen zu bessern.

Durchblutungsstörungen treten einmal als periphere arterille Verschlußkrankheit auf, bei der arterielle Blutgefäße durch Einlagerungen von Kalk und Cholesterin starr und eng werden. Als Konsequenz der daraus resultierenden Mangeldurchblutung kommt es insbesondere bei Belastung zu Schmerzen in den mit Sauerstoff unterversorgten Muskeln, zu Ulcera, Gangrän und Amputation. Diese Vorgänge können auch das Herz betreffen und führen dann zur Angina pectoris. Im Gehirm können Schwindel und Tinnitus Ausdruck einer Durchblutungsstörung sein. Aber auch durch weitere, bisher unbekannte Ursachen kann es zu Durchblutungsstörungen kommen. So gibt es das Raynaud-Syndrom und andere Durchblutungsstörungen der oberen Extremität und an anderen Lokalisationen, bei denen die Mangeldurchblutung zu Schmerzen und Unterversorgung des Gewebes bis hin zur Nekrose führt. Nach Transplantation gibt es Situationen, in denen das transplantierte Organ nach zunächst guter Versorgung immer schlechter durchblutet wird, bis es schließlich seine Funktion aufgibt.

Es ist bekannt, daß L-Arginin die Durchblutung der Beinarterie bei Normalprobanden erhöhen kann (R.H. Böger, S.M. Bode-Böger, D. Tsikas, J.C. Frölich, Intravenous L-arginine induces nitric oxide formation and increases peripheral arteriolar blood flow in humans, Endothelium 1993; 1 (Suppl.), S. 84). Dies wird in Zusammmenhang gebracht mit einer vermehrten Synthese von Stickstoffmonoxid (NO), welches ein Vasodilator ist. Es ist jedoch bekannt, daß einfache Vasodilatatoren wie Hydralazin die oben genannten Krankheitsbilder nicht positiv beeinflussen.

Der Erfindung liegt die Beobachtung zugrunde, daß L-Arginin nicht nur die Durchblutung bei Normalprobanden erhöht, sondern noch weitere Wirkungen entfaltet. Es wurde gefunden, daß die Verabfolgung von L-Arginin die Thrombozytenaggregation bei Normalprobanden hemmt und die Konzentration von cyclischen Guanosinmonophosphat (cGMP) erhöht (Beispiel 1).
Damit ist eine wesentliche Unterscheidung zu einer reinen Vasodilatation gegeben und eine Ähnlichkeit zu PGE₁ hergestellt, welches zur Zeit für die Behandlung der arteriellen Verschlußkrankheit eingesetzt wird und ebenfalls vasodilatierend und thrombozytenaggregationshemmend wirkt (J.R. Weeks, N. Chandra Sekhar, D.W. Ducharme. Relative activity of prostaglandin E1, A1, E2 und A2 on lipolysis, platelet aggregation, smooth, muscle and the cardiovascular system. J. Pharm. Pharmac., 21 (1969), 103-108).
Den Thrombozyten kommt bei der Entstehung und Fortentwicklung von Gefäßschäden organischer und funktioneller Art eine wichtige Rolle zu, weil sie nach ihrer Aktivierung eine große Anzahl von Mediatoren freisetzen. Darüber hinaus hat sich gezeigt, daß möglicherweise noch andere Mechanismen eine Rolle spielen können, die zwar im Tierversuch, jedoch noch nicht beim Menschen nachgewiesen sind. So kann L-Arginin die Ansammlung von Zellen im infarzierten Gewebe verhindern (K.Nakanishi, J. Vinten-Johansen, D.J. Lefer, Z. Zhao, W.C. Fowler, D.S. McGee, W.E. Johnston: Intracoronary L-arginine during reperfusion improves endothelial function and reduces infarct size. Am. J. Physiol., 263 /1992), H1650-H1658). Bei der Abstoßungsreaktion von Transplantaten kommt es gleichfalls zur Einwanderung von Zellen (Makrophagen, Granulozyten, Lymphozyten) sowie zur Ablagerung von Thrombozyten in den Gefäßwänden, die dadurch verengt werden.

L-Arginin hat gegenüber PGE₁ bei den genannten Indikationen erfindungsgemäß wesentliche Vorteile:
1. Bei der Infusion von L-Arginin gibt es keine lokale Rötung und Schmerzen. Diese unerwünschte Wirkung tritt bei PGE₁ oft auf und ist insbesondere bei para-venöser oder paraarterieller Infusion sehr schmerzhaft.
2. L-Arginin kann auch oral zugeführt werden und entfaltet dabei seine Wirkungen (W.E. Smoyer, B.H. Brouhard, D.K. Rassin, L. LaGrone: Enhanced GFR response to oral versus intravenous arginine administration in normal adults. J. Lab. Clin. Med. 118 (1991), 166-175). PGE₁ kann nach oraler Gabe nicht im Gefäßsystem wirksam werden, weil es im Magen-Darm-Trakt weitestgehend zerstört wird.

Diese unterschiedlichen Wirkungen wurden in eigenen Untersuchungen an Patienten mit peripheren Durchblutungsstörungen offenbar (Beispiel 2).

Entsprechend der vorliegenden Erfindung ist L-Arginin oder ein Prodrug, Salz oder sonstiges Derivat geeignet als Thrombozytenaggregation hemmes, insbesondere zur leerbesserung der Funktion und überlebenzeit von Transplantaten, zur Behandlung von Impotentia coeundi und Haarwuchsstörungen.

Die Herstellung von Arginin erfolgt nach allgemein bekannten Verfahren.
Die Herstellung von Arzneimitteln aus Arginin erfolgt nach bekannten Verfahren die Darreichungsformen zur parenteralen Gabe beinhalten gelöste Formen, in Liposomen zubereitete Formen und feste Formen mit Lösungsmittel. Zur oralen Gabe sind Tabletten, Kapseln und Zubereitungen mit retardierter Freisetzung geeignet. Desgleichen können Arginin-haltige Zubereitungen wie Salben, Cremes und Liquida zur lokalen Applikation, z.B. bei Ulcus cruris oder bei Haarwuchsstörungen, hergestellt werden.

Geeignete Formen des Arginins sind seine Salze wie Arginin-HCl, -aspartat, -nitrat, -phosphat etc.. Geeignete Formen des Arginins sind auch Substanzen, die als Prodrug erst im Körper zu Arginin metabolisiert werden, wie z.B. Citrullin.

Die nachstehenden Beispiele erläutern die Erfindung.

### Beispiel 1

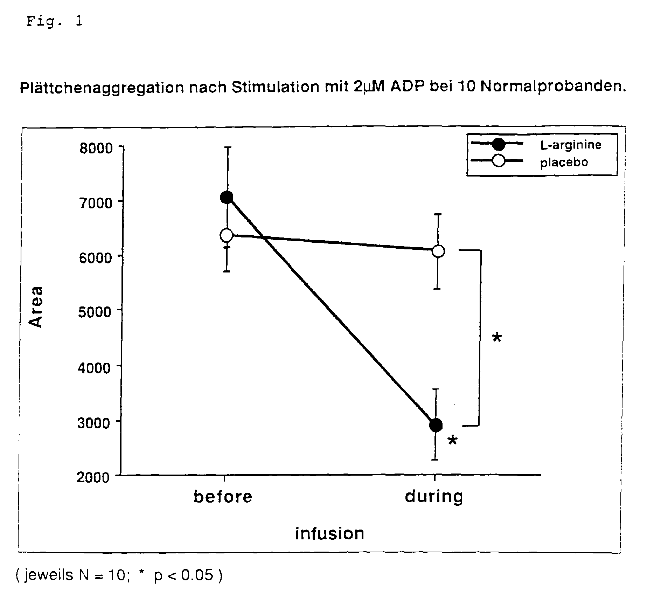

L-Arginin wurde in einer Dosis von 30 g an 10 Normalprobanden in Form einer 30minütigen intravenösen Infusion verabfolgt. Der Blutfluß in der A. femoralis wurde mit Hilfe eines Image-directed Duplex Ultrasound Systems (Diasonics Inc., CA. USA) erfaßt. Er stieg um 43,5 % an (p < 0,02). Dies war bedingt durch eine Zunahme der Flußgeschwindigkeit und nicht durch eine Zunahme des Gefäßdurchmessers. Die Ausscheidung von cGMP im Urin stieg im Vergleich zur Kontrollphase vor Beginn der Infusion um 65 % während der Arginin-Infusion an (p < 0,05). Während der Infusion des Lösungsmittels für Arginin, die an einen anderen Tag durchgeführt wurde, stieg die Konzentration von cGMP lediglich um 25,1 % an, während der Blutfluß in der A. femoralis unverändert blieb. Die Differenz der Ausscheidung von cGMP zwischen der Arginin- und der Lösungsmittelinfusion war statistisch signifikant (p < 0,05).
Vor der Infusion und am Ende der Infusion wurde thrombozytenreiches Plasma hergestellt und die Thrombozytenaggregation nach Born im Aggregometer nach Stimulation mit ADP (2 µM) bestimmt. Es zeigte sich, daß die Aggregation durch Arginin signifikant im Vergleich zu Lösungsmittel gehemmt wurde ( p < 0,05) (Fig. 1).

### Beispiel 2

Zwei Patienten mit peripherer arterieller Verschlußkrankheit beider Unterschenkel (Grad III nach Fontaine) erhielten eine intravenöse Infusion von 30 g L-Arginin an einem Tag und eine Infusion von 40 µg PGE₁ (Prostavasin®) an einem anderen Tag. Der Blutfluß in der A. femoralis stieg in beiden Fällen am Ende der Infusion um etwa 50 % an. Im Fall von PGE₁ kam es zu heftigen Schmerzen an der Infusionsstelle und im Verlauf der Vene. Arginin wurde ohne jede Nebenwirkung, ebenso wie durch die in Beispiel 1 zitierten 10 Normalprobanden, vertragen.

## Patentansprüche

1. Verwendung von L-Arginin zur Herstellung eines Arzneimittels als Thrombozytenaggregationshemmer.

2. Verwendung gemäß Anspruch 1 zur Herstellung eines Arzneimittels zur Behandlung von Impotentia coeundi.

3. Verwendung gemäß Anspruch 1 zur Herstellung eines Arzneimittels zur Verbesserung der Funktion und Überlebenszeit eines Transplantats.

4. Verwendung von L-Arginin zur Herstellung eines Arzneimittels zur Behandlung von Haarwuchsstörungen.

## Claims

1. Use of L-arginine in the manufacture of a pharmaceutical composition as a platelet aggregation inhibitor.

2. Use according to claim 1 in the manufacture of a pharmaceutical composition for the treatment of Impotentia coeundi.

3. Use according to claim 1 in the manufacture of a pharmaceutical composition to enhance the function and the survival time of a transplant.

4. Use of L-arginine in the manufacture of a pharmaceutical composition for the treatment of dysfunctional growth of hair.

## Revendications

1. Utilisation de L-arginine pour la préparation d'un médicament en tant qu'inhibiteur de l'agrégation plaquettaire.

2. Utilisation selon la revendication 1 pour la préparation d'un médicament pour le traitement de l'impuissance.

3. Utilisation selon la revendication 1 pour la préparation d'un médicament destiné à améliorer le fonctionnement et la durée de survie d'une greffe.

4. Utilisation de L-arginine pour la préparation d'un médicament visant à traiter les désordres de la croissance des cheveux.
